# EUROPÄISCHE PATENTSCHRIFT

(11) **EP 2 719 682 B1**
(45) Veröffentlichungstag und Bekanntmachung des Hinweises auf die Patenterteilung: **27.12.2017**
(21) Anmeldenummer: 13187473.7
(22) Anmeldetag: 07.10.2013
(51) Int. Cl.: C07C 201/08, C07C 201/16, C07C 205/06

(54) **Verfahren zur kontinuierlichen Herstellung von Nitrobenzol**
Method for continuous production of nitrobenzene
Procédé de fabrication continue de nitrobenzène

(30) Priorität: 10.10.2012 EP 12188027
(43) Veröffentlichungstag der Anmeldung: 16.04.2014
(73) Patentinhaber: Covestro Deutschland AG, 51373 Leverkusen (DE)
(72) Erfinder: Knauf, Thomas, 41542 Dormagen (DE); Münnig, Jürgen, 41564 Kaarst (DE); Schmiedler, Jörg, 47055 Duisburg (DE)
(74) Vertreter: Levpat

(56) Entgegenhaltungen:
- EP-B1- 1 816 117
- HOBBS F D ET AL: "Report 1 - Nitrobenzene", 1. Dezember 1980 (1980-12-01), DOCUMENT UNITED STATES ENVIRONMENTAL PROTECTION AGENCY, UNITED STATES ENVIRONMENTAL PROTECTION AGENCY, US, PAGE(S) 1 - 60, XP002693091, * Abschnitte III. "Process Descriptions", IV. "Emissions", V. "Applicable Control Systems", und Appendix D "Cost Estimate Details and Calculations" *
- D. PERRY, D. E. NUTTER, A. HALE: "Liquid Distribution for Optimum Packing Performance", CHEM. ENG. PROGRESS, Bd. 86, Januar 1990 (1990-01), Seiten 30-35, XP008166699,

## Beschreibung

Die vorliegende Erfindung betrifft ein Verfahren zur Herstellung von Nitrobenzol, bei dem die im Prozess anfallenden Abgasströme umfassend Benzol und (in Spuren) Nitrobenzol, Leicht- und Mittelsieder, ggf. nichtkondensierbare Gase sowie ggf. Wasser, optional nach Entfernung von Stickoxiden, in einer Absorptionskolonne mit Nitrobenzol, das nur sehr geringe Mengen (maximal 50 ppm) an Benzol enthält und das über einen Flüssigkeitsverteiler mit 50 bis 200 Tropfstellen pro Quadratmeter, bevorzugt 60 bis 120 Tropfstellen pro Quadratmeter, verteilt wird, gewaschen werden, wobei (i) ein flüssiger Strom umfassend Benzol und Nitrobenzol, ggf. organische Leicht- und Mittelsieder, im Fall von Schwefelsäure als Waschmittel zusätzlich umfassend Schwefelsäure, und (ii) an Benzol und ggf. organischen Leicht- und Mittelsiedern abgereichertes Abgas erhalten werden. Ein nach dem erfindungsgemäßen Verfahren gereinigtes Abgas eignet sich besonders für die Verbrennung in einer thermischen Abluftreinigung.

Nitrobenzol ist ein wichtiges Zwischenprodukt der chemischen Industrie, das insbesondere zur Herstellung von Anilin und damit zur Herstellung von Methylendiphenyldiisocyanat (MDI) und den darauf basierenden Polyurethanen benötigt wird.

Die Nitrierung von Benzol mit Salpetersäure zu einem Roh-Nitrobenzol war bereits Gegenstand zahlreicher Veröffentlichungen und Patentanmeldungen. Die heute gängigen Verfahren entsprechen im Wesentlichen dem Konzept der adiabaten Nitrierung von Benzol durch ein Gemisch von Schwefel- und Salpetersäure (sog. *Mischsäure).* Ein solches Verfahren wurde erstmals in US 2,256,999 beansprucht und ist in heutigen Ausführungsformen beispielsweise in EP 0 436 443 B1**,** EP 0 771 783 B1 und US 6 562 247 B2 beschrieben. Die Verfahren mit adiabater Reaktionsführung zeichnen sich insbesondere dadurch aus, dass keine technischen Maßnahmen ergriffen werden, um der Reaktionsmischung Wärme zu- oder abzuführen.

Auch sind isotherme Verfahren zur Nitrierung von Benzol mit Mischsäure beschrieben, wie beispielsweise in EP 0 156 199 B1 beschrieben.

Auch sind Verfahren zur Nitrierung von Benzol bekannt, die ohne den Einsatz von Schwefelsäure auskommen. Diese sind beispielsweise beschrieben in US 2 739 174 oder US 3 780 116.

Grundsätzlich sind auch Gasphasenverfahren zur Nitrierung von Benzol mit Salpetersäure oder Stickstoffoxiden möglich, jedoch sind die dadurch erzielbaren Ausbeuten noch gering (EP 0 078 247 B1**,** EP 0 552 130 B1).

Das Roh-Nitrobenzol weist als Verunreinigungen noch Wasser, Benzol sowie Nitrophenole und Dinitrobenzol und - sofern mit Mischsäure nitriert wurde - Schwefelsäure auf, die durch geeignete Aufarbeitungsverfahren wie z. B. Wasch- und Destillationsstufen abgetrennt werden. Eine mögliche Ausführungsform dieser Aufarbeitung ist in EP 1 816 117 B1 beschrieben, wo das Nitrobenzol einer sauren Wäsche, einer alkalischen Wäsche, einer neutralen Wäsche und abschließend einer destillativen Reinigung unterzogen wird.

Die Aufarbeitung des alkalischen Abwassers der alkalischen Wäsche ist in EP 1 593 654 A1 beschrieben, wobei in einem Verfahren der thermischen Druckzersetzung die Behandlung von aromatischen Nitroverbindungen enthaltenden Abwässern bei erhöhten Temperaturen und Druck durchgeführt wird. Die nach diesem Verfahren behandelten Abwässer können ohne Verdünnung direkt einer biologischen Kläranlage zugeführt werden.

Die in der Nitrierung entstehenden Stickoxide (NOₓ-Gase) können wie in US 5,313,009 beschrieben mittels Alkalilauge behandelt und als Natriumnitrat und -nitrit herausgewaschen werden. Zusätzlich wird auch noch Kohlendioxid, das im Nitrierprozess entsteht, als Natriumcarbonat gebunden.

In US 5,963,878 wird ein Verfahren offenbart, in dem von strategischen Bereichen des Nitriersystems NOₓ-Gase gewonnen, mit Luft und Wasser, beispielsweise in einer Einheit mit gepacktem Bett, bei höheren Temperaturen und unter Druck, in Berührung gebracht werden, wobei die NOₓ-Gase durch das Wasser unter Bildung schwacher Salpetersäure absorbiert werden. Die schwache Salpetersäure wird in den Reaktionsprozess zurückgeführt. Kohlendioxid wird in einem NOₓ-Gaswaschturm nicht absorbiert, wenn der Gaswaschturm in einem sauren Modus betrieben wird. Sauberes NOₓ-freies Abluftgas wird aus der Einheit mit gepacktem Bett in die Umwelt entlassen. All diesen Verfahren ist gemeinsam, dass eine weitere Behandlung der Abgase von Nitrieranlagen nicht vorgesehen ist.

In WO2012013678A2 wird beschrieben, dass zahlreiche Untersuchungen in der Vergangenheit darauf abzielten die Qualität des Roh-Nitrobenzols zu verbessern und somit die Ausbeute bezüglich Benzol und Salpetersäure zu erhöhen. Dank dieser Entwicklungen sind die heutigen adiabaten Flüssigphasenverfahren so ausgereift, dass es allen gelingt, ein Roh-Nitrobenzol herzustellen, das einen geringen Gehalt an Nebenprodukten aufweist, also nur zwischen 100 ppm und 300 ppm an Dinitrobenzol und zwischen 1500 ppm und 2500 ppm an Nitrophenolen enthält, wobei Pikrinsäure einen Anteil von 10 % bis 50 % an den Nitrophenolen annehmen kann.

Die Veröffentlichung ***"***Organic Chemical Manufacturing, Volume 7: Selected Processes" der US-amerikanischen Umweltbehörde (United States Environmental Protection Agency; EPA-450/3-80-028b, Dezember 1980) offenbart ein Verfahren zur Reinigung von Abgasen aus einem Nitrierprozess, bei dem die Abgase in einem Absorptionsturm mit Nitrobenzol gereinigt werden. Details zur Verteilung des Nitrobenzols in dem Absorptionsturm werden nicht genannt. Ferner wird lediglich offenbart, dass als Waschflüssigkeit Nitrobenzol aus dem sog. Nitrobenzol-Stripper verwendet werden könne. Details zur Reinheit dieses Nitrobenzols sind nicht angegeben.

DE-OS-29 21 487 offenbart ein Verfahren zur Entfernung von flüchtigen aromatischen Verbindungen aus ggf. noch Stickoxide und Salpetersäure enthaltenden Abgasen durch Behandlung der Abgase mit einem flüssigen Nitriermittel, bspw. einem Gemisch aus Salpeter- und Schwefelsäure. Die Verwendung von Nitrobenzol als Waschflüssigkeit wird nicht offenbart.

Beim Betreiben einer Nitrieranlage zur Herstellung von Nitrobenzol aus Benzol gemäß dem Stand der Technik kann festgestellt werden, dass die isolierte molare Menge an Endprodukt Nitrobenzol und an den verfahrensimmanenten Nebenprodukten Dinitrobenzol und Nitrophenol geringer ist als die entsprechende Menge an eingesetztem Benzol. Der Auslass für diesen Verlust ist offensichtlich das Abgas der Produktionsanlage, was aus ökologischer Sicht unerwünscht ist. Darüber hinaus ist der Verlust auch mit ökonomischen Nachteilen verbunden.

Es bestand daher ein Bedarf an einer Verbesserung der bestehenden Nitriertechnik dahingehend, dass die Umweltbelastung mit Organika über das Abgas verringert wird. Erstrebenswert war darüber hinaus, die Verringerung der Umweltbelastung möglichst so durchzuführen, dass sie mit wirtschaftlichen Vorteilen verbunden ist. Insbesondere war erstrebenswert, im Abgas mitgerissenes Benzol einer wirtschaftlichen Verwertung zuzuführen.

Dem vorstehend Gesagten Rechnung tragend ist ein Gegenstand der vorliegenden Erfindung ein Verfahren zur Herstellung von Nitrobenzol umfassend die Schritte
a) Nitrierung von Benzol mit Salpetersäure oder Gemischen aus Salpetersäure und Schwefelsäure, bevorzugt mit Gemischen aus Salpeter- und Schwefelsäure unter Einsatz eines stöchiometrischen Überschusses an Benzol bezogen auf Salpetersäure, und anschließende Phasentrennung in eine wässrige Phase und eine organische Phase, welche im Folgenden als Roh-Nitrobenzol bezeichnet wird,
b) Waschen des Roh-Nitrobenzols aus Schritt a) mit wässriger Waschflüssigkeit in mindestens drei Waschschritten und jeweils anschließender Abtrennung der Waschflüssigkeit, wobei nach Abtrennung der im der letzten Waschschritt eingesetzten Waschflüssigkeit ein vorgereinigtes Nitrobenzol erhalten wird, welches neben Nitrobenzol mindestens noch Benzol und Wasser enthält,
c) Abdestillieren von Benzol und Wasser aus dem vorgereinigten Nitrobenzol aus Schritt b) in einem Destillationsapparat, wobei gereinigtes Nitrobenzol erhalten wird,
   wobei
d) in den Schritten a) bis c) anfallende Abgasströme umfassend Benzol und (in Spuren) Nitrobenzol sowie Leicht- und Mittelsieder, ggf. nichtkondensierbare Gase und ggf. Wasser, optional nach Entfernung von Stickoxiden, in einer Absorptionskolonne mit Nitrobenzol, das einen Benzolgehalt von < 50 ppm, bevorzugt < 20 ppm, besonders bevorzugt < 10 ppm, jeweils bezogen auf die Gesamtmasse des Nitrobenzols, aufweist und das über einen Flüssigkeitsverteiler mit 50 bis 200 Tropfstellen pro Quadratmeter, bevorzugt 60 bis 120 Tropfstellen pro Quadratmeter, verteilt wird, im Gegenstrom gewaschen werden, wobei (i) ein flüssiger Strom umfassend Benzol und Nitrobenzol, Leicht- und Mittelsieder und (ii) an Benzol und ggf. organischen Leicht- und Mittelsiedern abgereichertes Abgas erhalten werden;
   wobei der in Schritt d) erhaltene flüssige Strom umfassend Benzol und Nitrobenzol
e) zur Gewinnung eines Gemisches aus Benzol und Nitrobenzol destilliert wird, wobei diese Destillation so betrieben wird, dass organische Leichtsieder von Benzol und Nitrobenzol abgetrennt werden und so ein Gemisch aus Benzol und Nitrobenzol erhalten wird, welches
f) in Schritt b) zurückgeführt wird.
Ein nach dem erfindungsgemäßen Verfahren erhaltenes in Schritt d) gereinigtes Abgas eignet sich besonders für die Verbrennung in einer thermischen Abluftreinigung.
Das Wort *"ein"* ist im Rahmen dieser Erfindung im Zusammenhang mit zählbaren Größen nur dann als Zahlwort zu verstehen, wenn dies ausdrücklich gesagt wird (z. B. durch den Ausdruck *"genau ein"*)*.* Bspw. schließt der Ausdruck *"eine Absorptionskolonne"* die Möglichkeit des Vorhandenseins mehrerer (in Reihe oder parallel geschalteter) Absorptionskolonnen nicht aus.
Als *Leichtsieder* werden im Rahmen dieser Erfindung alle Verbindungen und azeotrop siedende Gemische von Verbindungen bezeichnet, deren Siedepunkte unterhalb dem von Benzol bei Normaldruck (1013 mbar) liegen. Typische Leichtsieder sind n-Heptan, Dimethylcyclopentan, 3-Ethylpentan, Cyclohexan, die isomeren Dimethylpentane, n-Hexan, Cyclopentan und n-Pentan. Als *Mittelsieder* werden im Rahmen dieser Erfindung alle Verbindungen und azeotrop siedende Gemische von Verbindungen bezeichnet, deren Siedepunkte oberhalb dem von Benzol bei Normaldruck (1013 mbar), aber unterhalb dem von Nitrobenzol bei Normaldruck (1013 mbar), liegen. Typische Mittelsieder sind Trimethylcyclopentan, Methylcyclohexan, Ethylcyclopentan, Bicycloheptan und Oktan.
Als *nichtkondensierbare Gase* werden im Rahmen der vorliegenden Erfindungen solche Stoffe verstanden, die unter Normalbedingungen gasförmig vorliegen und mit großtechnisch üblichen Kondensatoren (Temperaturen bis -20 °C) bei Normaldruck nicht verflüssigt werden können. Typische Beispiele sind Stickoxide (NOx) und Kohlendioxid.

Nachstehend wird die Erfindung im Detail erläutert. Verschiedene Ausführungsformen sind dabei beliebig miteinander kombinierbar, solange sich für den Fachmann aus dem Kontext nicht eindeutig das Gegenteil ergibt.

**Schritt a)** des erfindungsgemäßen Verfahrens kann im Prinzip nach allen im Stand der Technik bekannten Verfahren zur Nitrierung von Benzol erfolgen. Die genaue Art des Nitrierverfahrens ist nicht erfindungswesentlich. Bevorzugt ist eine adiabate Prozessführung, bei der eine Mischung aus Salpeter- und Schwefelsäure mit einem stöchiometrischen Überschuss an Benzol bezogen auf Salpetersäure eingesetzt wird, wobei die im Überschuss eingesetzte Schwefelsäure nach der Reaktion vom Rohprodukt abgetrennt, aufkonzentriert und in die Nitrierung zurückgefahren wird ("Kreislaufschwefelsäure"). Auch das überschüssige Benzol wird in die Nitrierung zurückgeführt ("Rückbenzol"). Besonders bevorzugt ist eine Verfahrensführung, wie sie in DE 10 2008 048713 A1, insbesondere Absatz [0024], beschrieben ist. Eine isotherme Prozessführung ist aber grundsätzlich auch möglich.
In **Schritt b)** des erfindungsgemäßen Verfahrens können die einzelnen Waschschritte prinzipiell in beliebiger Reihenfolge durchgeführt werden. Bevorzugt ist jedoch die Reihenfolge
(1) Wäsche mit einer nicht-alkalischen wässrigen Waschflüssigkeit
(2) alkalische Wäsche mit einer wässrigen Basenlösung, bevorzugt einer Alkalimetall- oder Erdalkalimetallhydroxidlösung, besonders bevorzugt mit einer Natriumhydroxidlösung
(3) neutrale Wäsche mit Wasser.

In Schritt b) (1) werden die Säurereste (d. h. Schwefelsäure), die das rohe Nitrobenzol enthält, ausgewaschen; daher wird dieser Verfahrensschritt auch als *saure Wäsche* bezeichnet.

Besonders bevorzugt erfolgt Schritt b) nach der in den Absätzen [0008] bis [0012] von EP 1 816 117 B1 beschriebenen Vorgehensweise. Besonders bevorzugt wird in der neutralen Wäsche in Schritt b) eine Elektrophorese eingesetzt, wie sie in EP 1 816 117 B1, Absatz [0013] beschrieben ist. In **Schritt c**) des erfindungsgemäßen Verfahrens werden nicht umgesetztes Benzol und Restmengen an Wasser bevorzugt in einer Rektifizierkolonne von Nitrobenzol abdestilliert. In dieser Destillationsstufe fällt gereinigtes Nitrobenzol als Sumpfprodukt der Destillationskolonne an. Es ist darüber hinaus möglich und für viele Folgeanwendungen bevorzugt, dieses als Sumpfprodukt anfallende gereinigte Nitrobenzol noch weiter zu reinigen, in dem das Nitrobenzol selbst destilliert (verdampft und rekondensiert) wird. Auf diese Weise wird hochreines Nitrobenzol gewonnen. Für diesen Zweck wird als Destillationsapparat bevorzugt eine Rektifizierkolonne eingesetzt, d. h., ein Apparat, in dem mindestens eine theoretische Trennstufe realisiert wird und bei dem ein flüssiger Rücklauf oben in die Kolonne eingespeist wird. Bevorzugt wird das hochreine Nitrobenzol in einer Destillationskolonne über Kopf entnommen. Eine Seitenstromentnahme ist jedoch ebenfalls vorstellbar. Besonders bevorzugt ist diese Feinreinigung nach der in WO 2012/013678 A2 beschriebenen Vorgehensweise. In allen bisher geschilderten Verfahrensschritten fallen gasförmige Abgasströme an. Verfahrensabgase werden bevorzugt am Phasentrennapparat von Schritt a), der sauren, alkalischen und neutralen Wäsche in Schritt b), den Vorlagetanks für Kreislaufschwefelsäure und Rückbenzol (siehe oben, adiabate Prozessführung von Schritt a)), den Roh- und Reinnitrobenzoltanks und den Abwassertanks der Wäsche von Schritt b) entnommen und vor der Durchführung von **Schritt d)** bevorzugt vereinigt. Abgase werden bevorzugt auch in den Vakuumsystemen des Verdampfers zur Aufkonzentration der Kreislaufschwefelsäure (adiabate Prozessführung, siehe oben) und dem Destillationsapparat zur Reinigung von Nitrobenzol (Schritt c)) gewonnen. Kondensat der Vakuumsysteme wird bevorzugt in den Wäschen weiter benutzt. Die einzelnen Abgasströme enthalten in variierender Zusammensetzung Benzol, Nitrobenzol, Leicht- und Mittelsieder, ggf. nichtkondensierbare Gase (NOx, Kohlendioxid) sowie ggf. Wasser. Diese Abgasströme werden in **Schritt d**) in einer Absorptionskolonne gewaschen. Bevorzugt kann dieser Wäsche in der Absorptionskolonne ein **Schritt d.0**) vorausgehen, in welchem die Abgase aus den Schritten a) bis c) von Stickoxiden befreit werden, entweder getrennt oder bevorzugt nach Vereinigung der einzelnen Abgasströme zu einem Gesamtabgasstrom. Die Reinigung des Abgasstroms/der Abgasströme von Stickoxiden kann nach allen aus dem Stand der Technik bekannten Verfahren erfolgen. Bevorzugt ist der Einsatz eines NOx-Absorbers wie in US 5,963,878, insbesondere in Spalte 2, Z. 12 bis Spalte 3, Z. 27, beschrieben. Alternativ kann eine ggf. erforderliche Stickoxidabtrennung auch in einem Schritt d) nachgelagerten **Schritt d**.**1**) erfolgen, der, wie zuvor für **Schritt d.0**) beschrieben, ausgestaltet ist. Dies ist besonders bevorzugt, um die Explosionsgrenzen des Abgasgemisches Benzol / Sauerstoff, die beim Einleiten von Sauerstoff in den Abgasstrom zur NOx-Absorption entstehen würden, zu vermeiden.

In Schritt d) des erfindungsgemäßen Verfahrens wird/werden der/die, ggf. von in Schritt d.0) erhaltene(n) von Stickoxiden befreite(n), Abgasstrom/Abgasströme in einer Absorptionskolonne mit Nitrobenzol geringen Benzolgehalts gewaschen, um mitgerissenes Benzol sowie ggf. weitere organische Bestandteile (Leicht- und Mittelsieder) auszuwaschen. Unabhängig davon, ob Schritt d.0) ausgeführt wird oder nicht, ist es bevorzugt, alle Abgasströme vor der Wäsche in Schritt d) zu einem einzigen Gesamtabgasstrom zu vereinigen. Sofern die Abgasströme aus den Schritten a) bis c) nicht bereits vor Schritt d.0) vereinigt wurden, werden bevorzugt alle aus Schritt d.0) erhaltenen von Stickoxiden befreiten Abgasströme vereinigt, bevor der Waschschritt d) durchgeführt wird.

Bevorzugt wird der Druck in der in Schritt d) eingesetzten Absorptionskolonne so gewählt, dass sich Benzol und Leicht- und Mittelsieder optimal auswaschen lassen. Die Erfindung betrifft demnach insbesondere auch ein Verfahren, bei dem Schritt d) bei einem absoluten Druck von 900 mbar bis 980 mbar, bevorzugt von 920 mbar bis 960 mbar, betrieben wird. Dabei wird der Druck bevorzugt im Eingang der Absorptionskolonne gemessen.

Ein wichtiges Kriterium für den als Waschflüssigkeit in Schritt d) einzusetzenden Nitrobenzolstrom ist dessen Benzolgehalt. Erfindungsgemäß wird solches Nitrobenzol als Waschflüssigkeit eingesetzt, das einen (per Gaschromatographie bestimmten) Benzolgehalt von < 50 ppm, bevorzugt von < 20 ppm, besonders bevorzugt von < 10 ppm, jeweils bezogen auf die Gesamtmasse des Nitrobenzols, aufweist.

Bevorzugt wird die Temperatur des Waschnitrobenzols in der Absorptionskolonne in Schritt e) so gewählt, dass sich Benzol und Leicht- und Mittelsieder optimal mit Nitrobenzol auswaschen lassen. Die Erfindung betrifft demnach insbesondere auch ein Verfahren, bei dem das als Waschflüssigkeit in Schritt d) eingesetzte Nitrobenzol aus Schritt c) eine Temperatur von 20 °C bis 75 °C, bevorzugt 25 °C bis 60 °C, besonders bevorzugt von 30 °C bis 45 °C, hat. Die Temperatur des Waschnitrobenzols wird dabei bevorzugt in der zuführenden Rohrleitung vor der Absorptionskolonne gemessen. Die Brüdentemperatur am Ausgang der Absorptionskolonne beträgt bevorzugt 20 °C bis 75 °C, besonders bevorzugt 20 °C und 50 °C, ganz besonders bevorzugt 20 °C bis 40°C und wird in der Brüdenleitung gemessen.

Erfindungsgemäß wird in Schritt d) ein Apparat zur Direktkondensation eingesetzt, in dem Benzol und Leicht- und Mittelsieder aus dem Abgas mit Waschnitrobenzol absorbiert werden und anschließend das so erhaltene Gemisch umfassend Nitrobenzol, Benzol und Leicht- und Mittelsieder aus der Absorptionskolonne ausgetragen wird. Die erfindungsgemäße Vorgehensweise ermöglicht nicht nur die effektive Entfernung von Benzol aus dem Abgas, sondern führt darüber hinaus zu einer starken Reduzierung des Gehalts an Leicht- und Mittelsiedern im Abgas, insbesondere an aliphatischen organischen Verbindungen. Dies wird erreicht durch den Einsatz eines Flüssigkeitsverteilers mit einer Tropfstellendichte von 50 bis 200 Tropfstellen pro Quadratmeter, bevorzugt von 60 bis 120 Tropfstellen pro Quadratmeter. Eine zu große Anzahl an Tropfstellen bedeutet einen zu großen Mehrbedarf an Absorptionsmittel, was zu einem erhöhten Kreislauf in der Rektifikationskolonne führt. Eine zu geringe Anzahl an Tropfstellen führt zu einem zu geringem Wirkungsgrad der Absorption. Ferner ist der äußerst geringe Gehalt an Benzol im Waschnitrobenzol wesentlich. Der erfindungsgemäße Benzolgehalt von < 50 ppm, bevorzugt < 20 ppm und besonders bevorzugt < 10 ppm ist geringer als die Spezifikation des für die Anilinproduktion (dem Hauptanwendungsgebiet von Nitrobenzol) üblicherweise verwendeten Nitrobenzols zulässt. Bevorzugt wird als Waschflüssigkeit gereinigtes Nitrobenzol aus Schritt c) verwendet. In diesem Fall ist Schritt c) so auszugestalten, dass die erfindungsgemäßen Anforderungen an den Restbenzolgehalt des Nitrobenzols eingehalten werden. Dies wird bevorzugt erreicht, indem die Destillation in Schritt c) bei einem absoluten Druck am Kopf der Destillationskolonne von 220 mbar bis 480 mbar, bevorzugt von 270 mbar bis 430 mbar, besonders bevorzugt von 320 mbar bis 380 mbar und bei einer Temperatur im Sumpf der Destillationskolonne von 100 °C bis 200 °C, bevorzugt von 120 °C bis 190 °C, besonders bevorzugt von 160 °C bis 180 °C, durchgeführt wird. Bevorzugt verfügt die in Schritt c) eingesetzte Destillationskolonne über einen Umlaufverdampfer, in dem der Destillationssumpf durch Beheizung mit Dampf auf die genannten Temperaturwerte erhitzt wird. Durch diese Maßnahmen wird ferner erreicht, dass der Restwassergehalt des Nitrobenzols bei < 100 ppm, bevorzugt bei < 70 ppm, besonders bevorzugt bei < 40 ppm, jeweils bezogen auf die Gesamtmasse des Nitrobenzols, liegt.

Geeignete Apparate werden bspw. beschrieben in Reinhard Billet; "Verdampfung und ihre technischen Anwendungen"; Verlag Chemie Weinheim - Deerfield Beach, Florida - Basel; 1981, Kapitel 4.1.2, Seite 208 bis 230.

Der in Schritt d) erhaltene flüssige Strom umfassend Benzol und Leicht- und Mittelsieder sowie Waschflüssigkeit, bevorzugt gereinigtes Nitrobenzol aus Schritt c), wird aufgearbeitet. Für diese Aufarbeitung ist eine Destillation in der die organischen Leichtsieder von Benzol und Nitrobenzol abgetrennt werden, vorgesehen. Die Leichtsieder werden verbrannt oder wieder in einen petrochemischen Prozess zurückgeführt. Das Gemisch von Nitrobenzol und Benzol wird in den Nitrierprozess zurückgeführt und zwar in die Wäsche des Rohnitrobenzols in Schritt b), bevorzugt in die saure Wäsche (Schritt b) (1)) des Rohnitrobenzols. In der vorliegenden Erfindung wird daher der in Schritt d) erhaltene flüssige Strom umfassend Benzol und Nitrobenzol
e) zur Gewinnung eines Gemisches aus Benzol und Nitrobenzol, destilliert.

Die Destillation in Schritt e) wird so betrieben, dass ein Gemisch aus Benzol und Nitrobenzol erhalten wird, welches
f) in Schritt b), bevorzugt in die erste Waschstufe von Schritt b), zurückgeführt wird.

Eine bevorzugte Ausführungsform zum Betreiben einer Absorptionskolonne mit einer mehrstufigen Kondensation wird im Folgenden beschrieben:
Alle Abgase des Betriebes werden gesammelt und druckgeregelt einer Abgaswäsche, die sowohl im Über- als auch im Unterdruck betrieben werden kann, zugeführt. Das Abgas enthält Stickoxide, Benzol, weitere organische Bestandteile wie Leicht- und Mittelsieder und geringe Mengen an Nitrobenzol sowie Stickstoff aus Druckhaltungen der Nitrieranlage und Tank-Inertisierungen.

Das Leichtsieder, Benzol, Nitrobenzol und Mittelsieder, die zwischen Benzol und Nitrobenzol sieden, sowie Stickoxide enthaltende Abgas wird bei leicht vermindertem Druck von 900 mbar bis 1000 mbar von unten in die Abgaswäsche geleitet. Das Abgas hat eine Temperatur von 20 °C bis 75 °C. Die Abgaswäsche, die als Direktkondensation ausgeführt ist, wird mit gereinigtem Nitrobenzol aus Schritt c) betrieben, welches über einen Flüssigkeitsverteiler mit 50 bis 200 Tropfstellen pro Quadratmeter, bevorzugt 60 bis 120 Tropfstellen pro Quadratmeter, verteilt wird. Das gereinigte Nitrobenzol, welches eine Temperatur von 20 °C bis 75 °C hat, kondensiert Leichtsieder, Benzol, Mittelsieder und Spuren an Nitrobenzoldämpfen aus dem Abgas. Dabei wird das Reinnitrobenzol über eine Einstoffdüse im Gegenstrom mit einem Tropfenspektrum von 0,2 mm bis 2,0 mm verdüst. Der Stoffaustausch wird über eine Strukturpackung, alternativ Füllkörperfüllung, gewährleistet. Die Verweilzeit des Gasstromes in der Absorptionskolonne beträgt kleiner 30 Sekunden. Die so erzeugte Austauschoberfläche reicht also aus, um eine effektive Absorption der Abgase zu erzielen. Es werden 0,5 bis 5 Tonnen pro Stunde Flüssigphase aus der Absorptionskolonne, enthaltend Nitrobenzol, Mittelsieder, Benzol, Leichtsieder und Spuren an Stickoxid und Stickstoff, in die Wäsche nach Schritt b) (1) (saure Wäsche) zurückgeführt. Es werden dadurch ca. 10 bis 50 kg pro Stunde an Benzol zurückgewonnen. Erfindungsgemäß wird die Flüssigphase, bevor sie der sauren Wäsche zugeführt wird, destilliert, um unerwünschte Leichtsieder zu entfernen.

Das so gereinigte Abgas kann problemlos in eine thermische Abluftreinigung geführt werden.

### Beispiele

In allen Beispielen erfolgte die Analytik des Abgasstromes mittels Gaschromatographie. Die Identifizierung der Organika erfolgte via GC-MS (Massenspektrometrie).

### Beispiel 1 (Vergleichsbeispiel): unbehandeltes Abgas

Gemäß den Schritten a) bis b) des erfindungsgemäßen Verfahrens wurde zunächst vorgereinigtes Nitrobenzol hergestellt und dieses in Schritt c) in einer Rektifizierkolonne von Benzol, Wasser und Leichtsiedern befreit. Das gereinigte Nitrobenzol wird als Sumpfprodukt entnommen. Das dabei in der Nitrieranlage und den zugehörigen Tankbehältern anfallende Abgas wird in einem mit Wasser betriebenen Abgastauchsystem gesammelt und anschließend einer thermischen Abluftreinigung zugeführt. Die Nitrieranlage wird mit einer Produktionslast von 42 Tonnen Nitrobenzol pro Stunde gefahren. Der Abgasstrom beträgt ca. 150 m³ pro Stunde und enthält ca. 160 g/m³ Benzol . Die Verluste an Benzol über das ausgetragene Abgas betragen 24 kg Benzol pro Stunde. Dies sind 0,09 % des eingesetzten Benzols.

### Beispiel 2 (Vergleichsbeispiel): Entfernung von Benzol aus dem Abgas mit Wasser in einer Abgaswäsche mit einstufiger Kondensation

Gemäß den Schritten a) bis b) des erfindungsgemäßen Verfahrens wurde zunächst vorgereinigtes Nitrobenzol hergestellt und dieses in Schritt c) in einer Rektifizierkolonne von Benzol, Wasser und Leichtsiedern befreit. Das gereinigte Nitrobenzol wird als Sumpfprodukt entnommen. Das dabei in der Nitrieranlage und den zugehörigen Tankbehältern anfallende Abgas wird in einem mit Wasser betriebenen Abgastauchsystem gesammelt und anschließend in einen wie oben beschriebenen Abgaswäscher gefahren. Die Nitrieranlage wird mit einer Produktionslast von 42 Tonnen Nitrobenzol pro Stunde gefahren. Der Abgasstrom beträgt ca. 150 m³ pro Stunde und enthält ca. 160 g/m³ Benzol. Die im Abgas befindlichen Organika werden mit Wasser, das aus der sauren Wäsche stammt und eine Temperatur von 40 °C hat, niedergeschlagen,. Die verwendete Wassermenge beträgt 2 Tonnen pro Stunde. Das Wasser wird verdüst und über einen Flüssigkeitsverteiler mit 80 Tropfstellen pro Quadratmeter verteilt. Es werden 3 kg Benzol pro Stunde zurückgewonnen. Das so behandelte Abgas, welches noch ca. 140 g/m³ an Benzol enthält, wird einer thermischen Abluftreinigung zugeführt. Die Verluste an Benzol über die Abgasschiene betragen 21 kg Benzol pro Stunde. Dies sind 0,079 % des eingesetzten Benzols.

### Beispiel 3 (Vergleichsbeispiel): Entfernung von Benzol aus dem Abgas mit Roh-Nitrobenzol in einer Abgaswäsche mit einstufiger Kondensation

Gemäß den Schritten a) bis b) des erfindungsgemäßen Verfahrens wurde zunächst vorgereinigtes Nitrobenzol hergestellt und dieses in Schritt c) in einer Rektifizierkolonne von Leichtsiedern befreit. Das gereinigte Nitrobenzol wird als Sumpfprodukt entnommen. Das dabei in der Nitrieranlage und den zugehörigen Tankbehältern anfallende Abgas wird in einem mit Wasser betriebenen Abgastauchsystem gesammelt und anschließend in einen wie oben beschriebenen Abgaswäscher gefahren. Die Nitrieranlage wird mit einer Produktionslast von 42 Tonnen pro Stunde gefahren. Der Abgasstrom beträgt 150 m³ pro Stunde und enthält ca. 160 g/m³ Benzol. Die im Abgas befindlichen Organika werden mit Roh-Nitrobenzol, das aus dem Roh-Nitrobenzoltank stammt und 7,2 Massen-% Benzol enthält und eine Temperatur von 36 °C hat, niedergeschlagen. Die verwendete Menge an Rohnitrobenzol beträgt 1,1 Tonnen pro Stunde. Das Rohnitrobenzol wird verdüst und über einen Flüssigkeitsverteiler mit 80 Tropfstellen pro Quadratmeter verteilt. Nach dem Abgaswäscher wurde mehr Benzol (ca. 240 g/m³) pro Stunde im Abgas gefunden als vor dem Abgaswäscher. Das so behandelte Abgas wird einer thermischen Abluftreinigung zugeführt.

### Beispiel 4 (Vergleichsbeispiel): Entfernung von Benzol aus dem Abgas mit gereinigtem Nitrobenzol in einer Abgaswäsche mit einer einstufigen Absorptionskolonne

Gemäß den Schritten a) bis b) des erfindungsgemäßen Verfahrens wurde zunächst vorgereinigtes Nitrobenzol hergestellt und dieses in Schritt c) in einer Rektifizierkolonne von Leichtsiedern befreit. Das gereinigte Nitrobenzol wird als Sumpfprodukt entnommen. Das dabei in der Nitrieranlage und den zugehörigen Tankbehältern anfallende Abgas wird in einer Abgassammelleitung gesammelt und in einen Abgaswäscher gefahren. Die Nitrieranlage wird mit einer Produktionslast von 42 Tonnen pro Stunde gefahren. Der Abgasstrom beträgt ca. 150 m³ pro Stunde und enthält ca. 160 g/m³ Benzol. Die im Abgas befindlichen Organika werden mit gereinigtem Nitrobenzol mit einem Gehalt von 100 ppm an Benzol, das aus dem "Nitrobenzoltank rein" stammt und eine Temperatur von 38 °C hat, niedergeschlagen. Es werden 1,1 Tonnen gereinigtes Nitrobenzol pro Stunde verdüst und über einen Flüssigkeitsverteiler mit 50 Tropfstellen pro Quadratmeter verteilt. Es werden 13,5 kg Benzol pro Stunde zurückgewonnen. Das so behandelte Abgas wird einer thermischen Abluftreinigung zugeführt. Die Verluste an Benzol über die thermischen Abluftreinigung betragen 10,5 kg Benzol pro Stunde. Dies sind 0,039 % des eingesetzten Benzols.

**Tabelle 1: Analyse des Abgasstromes vor und nach dem Abgaswäscher in Beispiel 4:**

| Stoff | Nitrobenzol | Benzol |
|---|---|---|
| Menge vor Abgaswäscher [g/m³ Abgas] | 0,021 | 160 |
| Menge nach Abgaswäscher [g/m³ Abgas] | 0,132 | 71 |

Das nach der Abgaswäsche im Abgas befindliche Nitrobenzol wird in einer NOx-Absorptionsstufe (Schritt d.1)) zum Großteil aus dem Abgas entfernt.

### Beispiel 5 (erfindungsgemäß): Entfernung von Benzol aus dem Abgas mit gereinigtem Nitrobenzol in einer Abgaswäsche mit einer einstufigen Absorptionskolonne

Gemäß den Schritten a) bis b) des erfindungsgemäßen Verfahrens wurde zunächst vorgereinigtes Nitrobenzol hergestellt und dieses in Schritt c) in einer Rektifizierkolonne von Leichtsiedern befreit. Das gereinigte Nitrobenzol wird als Sumpfprodukt entnommen. Das dabei in der Nitrieranlage und den zugehörigen Tankbehältern anfallende Abgas wird in einer Abgassammelleitung gesammelt und in einen Abgaswäscher gefahren. Die Nitrieranlage wird mit einer Produktionslast von 42 Tonnen pro Stunde gefahren. Der Abgasstrom beträgt ca. 150 m³ pro Stunde und enthält ca. 160 g/m³ Benzol. Die im Abgas befindlichen Organika werden mit gereinigtem Nitrobenzol mit einem Gehalt von 5 ppm an Benzol, das aus dem "Nitrobenzoltank rein" stammt und eine Temperatur von 38 °C hat, niedergeschlagen. Es werden 1,1 Tonnen gereinigtes Nitrobenzol pro Stunde verdüst und über einen Flüssigkeitsverteiler mit 50 Tropfstellen pro Quadratmeter verteilt. Es werden 23,5 kg Benzol pro Stunde zurückgewonnen. Das so behandelte Abgas wird einer thermischen Abluftreinigung zugeführt. Die Verluste an Benzol über die thermischen Abluftreinigung betragen 0,5 kg Benzol pro Stunde. Dies sind 0,00187 % des eingesetzten Benzols.

**Tabelle 2: Analyse des Abgasstromes vor und nach dem Abgaswäscher in Beispiel 5:**

| Stoff | Nitrobenzol | Benzol |
|---|---|---|
| Menge vor Abgaswäscher [g/m³ Abgas] | 0,021 | 160 |
| Menge nach Abgaswäscher [g/m³ Abgas] | 0,132 | 2 |

Das nach der Abgaswäsche im Abgas befindliche Nitrobenzol wird in einer NOx-Absorptionsstufe (Schritt d.1)) zum Großteil aus dem Abgas entfernt.

### Beispiel 6 (erfindungsgemäß): Entfernung von Benzol aus dem Abgas mit gereinigtem Nitrobenzol in einer Abgaswäsche mit einer sechsstufigen Absorptionskolonne

Gemäß den Schritten a) bis b) des erfindungsgemäßen Verfahrens wurde zunächst vorgereinigtes Nitrobenzol hergestellt und dieses in Schritt c) in einer Rektifizierkolonne von Leichtsiedern befreit. Das gereinigte Nitrobenzol wird als Sumpfprodukt entnommen. Das dabei in der Nitrieranlage und den zugehörigen Tankbehältern anfallende Abgas wird in einer Abgassammelleitung gesammelt und in einen Abgaswäscher gefahren. Die Nitrieranlage wird mit einer Produktionslast von 42 Tonnen Nitrobenzol pro Stunde gefahren. Der Abgasstrom beträgt ca. 150 m³ pro Stunde und enthält ca. 160 g/m³ Benzol. Die im Abgas befindlichen Organika werden mit gereinigtem Nitrobenzol mit einem Gehalt von 5 ppm an Benzol, das aus dem "Nitrobenzoltank rein" stammt und eine Temperatur von 39 °C hat, in einem Abgaswäscher (Absorptionskolonne) mit sechsstufiger Kondensation niedergeschlagen. Es werden 1,1 Tonnen Nitrobenzol pro Stunde verdüst und über einen Flüssigkeitsverteiler mit 120 Tropfstellen pro Quadratmeter verteilt. Es werden 23,99 kg Benzol pro Stunde zurückgewonnen. Das so behandelte Abgas wird einer thermischen Abluftreinigung zugeführt. Die Verluste an Benzol über die thermischen Abluftreinigung betragen 0,01 kg Benzol pro Stunde. Dies sind 0,000037 % des eingesetzten Benzols.

Dieses Beispiel zeigt, dass bei gleicher Reinheit des eingesetzten Waschnitrobenzols die Ausgestaltung des Flüssigkeitsverteilers von großer Bedeutung für die Reduzierung des Benzolgehalts im Abgas ist.

**Tabelle 3: Analyse des Abgasstromes vor und nach dem Organika-Wäscher in Beispiel 6:**

| Stoff | Nitrobenzol | Benzol |
|---|---|---|
| Menge vor Abgaswäscher [g/m³ Abgas] | 0,019 | 160 |
| Menge nach Abgaswäscher [g/m³ Abgas] | 0,129 | 0,06 |

## Patentansprüche

1. Verfahren zur Herstellung von Nitrobenzol umfassend die Schritte
a) Nitrierung von Benzol mit Salpetersäure oder Gemischen aus Salpetersäure und Schwefelsäure und anschließende Phasentrennung in eine wässrige Phase und Roh-Nitrobenzol,
b) Waschen des Roh-Nitrobenzols aus Schritt a) mit wässriger Waschflüssigkeit in mindestens drei Waschschritten und jeweils anschließender Abtrennung der Waschflüssigkeit, wobei nach Abtrennung der im der letzten Waschschritt eingesetzten Waschflüssigkeit ein vorgereinigtes Nitrobenzol erhalten wird, welches neben Nitrobenzol mindestens noch Benzol und Wasser enthält,
c) Abdestillieren von Benzol und Wasser aus dem vorgereinigten Nitrobenzol aus Schritt b) in einem Destillationsapparat, wobei gereinigtes Nitrobenzol erhalten wird,
**dadurch gekennzeichnet, dass**
d) in den Schritten a) bis c) anfallende Abgasströme umfassend Benzol und Nitrobenzol in einer Absorptionskolonne mit Nitrobenzol, das einen Benzolgehalt von < 50 ppm, bezogen auf die Gesamtmasse des Nitrobenzols, aufweist und das über einen Flüssigkeitsverteiler mit 50 bis 200 Tropfstellen pro Quadratmeter verteilt wird, im Gegenstrom gewaschen werden, wobei (i) ein flüssiger Strom umfassend Benzol und Nitrobenzol und (ii) an Benzol abgereichertes Abgas erhalten werden;
wobei der in Schritt d) erhaltene flüssige Strom umfassend Benzol und Nitrobenzol
e) zur Gewinnung eines Gemisches aus Benzol und Nitrobenzol destilliert wird, wobei diese Destillation so betrieben wird, dass organische Leichtsieder - das sind Verbindungen und azeotrop siedende Gemische von Verbindungen, deren Siedepunkte unterhalb dem von Benzol bei Normaldruck (1013 mbar) liegen -, von Benzol und Nitrobenzol abgetrennt werden und so ein Gemisch aus Benzol und Nitrobenzol erhalten wird, welches
f) in Schritt b) zurückgeführt wird.

2. Verfahren nach Anspruch 1, bei dem Schritt d) bei einem absoluten Druck von 900 mbar bis 980 mbar betrieben wird.

3. Verfahren nach einem der Ansprüche 1 oder 2, bei dem als Waschflüssigkeit in Schritt d) gereinigtes Nitrobenzol aus Schritt c) eingesetzt wird.

4. Verfahren nach Anspruch 3, bei dem die Destillation in Schritt c) bei einem absoluten Druck am Kopf der Destillationskolonne von 220 mbar bis 480 mbar und bei einer Temperatur im Sumpf der Destillationskolonne von 100 C bis 200 °C betrieben wird.

5. Verfahren nach Anspruch 3, bei dem die Destillation in Schritt c) bei einem absoluten Druck am Kopf der Destillationskolonne von 270 mbar bis 430 mbar und bei einer Temperatur im Sumpf der Destillationskolonne von 120 C bis 190 °C betrieben wird, und bei dem das in Schritt d) als Waschflüssigkeit eingesetzte gereinigte Nitrobenzol einen Benzolgehalt von < 20 ppm, bezogen auf die Gesamtmasse des gereinigten Nitrobenzols, aufweist.

6. Verfahren nach Anspruch 3, bei dem die Destillation in Schritt c) bei einem absoluten Druck am Kopf der Destillationskolonne von 320 mbar bis 380 mbar und bei einer Temperatur im Sumpf der Destillationskolonne von 160 C bis 180 °C betrieben wird, und bei dem das in Schritt d) als Waschflüssigkeit eingesetzte gereinigte Nitrobenzol einen Benzolgehalt von < 10 ppm, bezogen auf die Gesamtmasse des gereinigten Nitrobenzols, aufweist.

7. Verfahren nach einem der Ansprüche 3 bis 6, bei dem das als Waschflüssigkeit in Schritt d) eingesetzte Nitrobenzol aus Schritt c) eine Temperatur von 20 °C bis 75 °C hat.

8. Verfahren nach einem der Ansprüche 1 bis 7, bei dem die Absorptionskolonne so betrieben wird, dass die sie verlassenden gasförmigen Brüden eine Temperatur von 20 °C bis 75 °C haben.

9. Verfahren nach einem der Ansprüche 1 bis 8, bei dem der in Schritt d) eingesetzte Flüssigkeitsverteiler 60 bis 120 Tropfstellen pro Quadratmeter hat.

## Claims

1. Process for preparing nitrobenzene, which comprises the steps
a) nitration of benzene by means of nitric acid or mixtures of nitric acid and sulphuric acid and subsequent phase separation into an aqueous phase and crude nitrobenzene,
b) washing of the crude nitrobenzene from step a) with aqueous washing liquid in at least three washing steps and in each case subsequent removal of the washing liquid, with a prepurified nitrobenzene containing nitrobenzene together with at least benzene and water being obtained after the washing liquid used in the last washing step has been separated off,
c) distillation of benzene and water from the prepurified nitrobenzene from step b) in a distillation apparatus, giving purified nitrobenzene,
**characterized in that**
d) gas streams comprising benzene and nitrobenzene which are obtained in steps a) to c) are scrubbed in counter current in an absorption column by means of nitrobenzene which has a benzene content of < 50 ppm, based on the total mass of the nitrobenzene, and is distributed by means of a liquid distributor having 50 to 200 dropping points per square meter, giving (i) a liquid stream comprising benzene and nitrobenzene and (ii) offgas depleted in benzene;
where the liquid stream comprising benzene and nitrobenzene which is obtained in step d)
e) is distilled to isolate a mixture of benzene and nitrobenzene, with this distillation being operated so that organic low boilers, namely compounds and azeotropically boiling mixtures of compounds whose boiling points are below that of benzene at atmospheric pressure (1013 mbar), are separated off from benzene and nitrobenzene so as to give a mixture of benzene and nitrobenzene which
f) is recirculated to step b).

2. Process according to Claim 1, wherein step d) is operated at an absolute pressure of from 900 mbar to 980 mbar.

3. Process according to either of Claims 1 and 2, wherein purified nitrobenzene from step c) is used as scrubbing liquid in step d).

4. Process according to Claim 3, wherein the distillation in step c) is operated at an absolute pressure at the top of the distillation column of from 220 mbar to 480 mbar and at a temperature at the bottom of the distillation column of from 100ºC to 200ºC.

5. Process according to Claim 3, wherein the distillation in step c) is operated at an absolute pressure at the top of the distillation column of from 270 mbar to 430 mbar and at a temperature at the bottom of the distillation column of from 120ºC to 190ºC and the purified nitrobenzene used as scrubbing liquid in step d) has a benzene content of < 20 ppm, based on the total mass of the purified nitrobenzene.

6. Process according to Claim 3, wherein the distillation in step c) is operated at an absolute pressure at the top of the distillation column of from 320 mbar to 380 mbar and at a temperature at the bottom of the distillation column of from 160ºC to 180ºC and the purified nitrobenzene used as scrubbing liquid in step d) has a benzene content of < 10 ppm, based on the total mass of the purified nitrobenzene.

7. Process according to any of Claims 3 to 6, wherein the nitrobenzene from step c) which is used as scrubbing liquid in step d) has a temperature of from 20ºC to 75ºC.

8. Process according to any of Claims 1 to 7, wherein the absorption column is operated in such a way that the gaseous vapours leaving the column have a temperature of from 20ºC to 75ºC.

9. Process according to any of Claims 1 to 8, wherein the liquid distributor used in step d) has from 60 to 120 dropping points per square metre.

## Revendications

1. Procédé de fabrication de nitrobenzène comprenant les étapes suivantes :
a) la nitration de benzène avec de l'acide nitrique ou des mélanges d'acide nitrique et d'acide sulfurique, puis la séparation de phases en une phase aqueuse et du nitrobenzène brut,
b) le lavage du nitrobenzène brut de l'étape a) avec un liquide de lavage aqueux en au moins trois étapes de lavage, puis à chaque fois la séparation du liquide de lavage, un nitrobenzène pré-purifié étant obtenu après la séparation du liquide de lavage utilisé dans la dernière étape de lavage, qui contient en plus du nitrobenzène au moins encore du benzène et de l'eau,
c) l'élimination par distillation du benzène et de l'eau du nitrobenzène pré-purifié de l'étape b) dans un appareil de distillation, du nitrobenzène purifié étant obtenu,
**caractérisé en ce que**
d) les courants de gaz d'échappement formés lors des étapes a) à c), comprenant du benzène et du nitrobenzène, sont lavés à contre-courant dans une colonne d'absorption avec du nitrobenzène, qui présente une teneur en benzène < 50 ppm, par rapport à la masse totale du nitrobenzène, et qui est distribué par un distributeur de liquide comprenant 50 à 200 emplacements d'égouttement par mètre carré, (i) un courant liquide comprenant du benzène et du nitrobenzène et (ii) un gaz d'échappement appauvri en benzène étant obtenus ;
le courant liquide obtenu à l'étape d) comprenant du benzène et du nitrobenzène,
e) une distillation est effectuée pour obtenir un mélange de benzène et de nitrobenzène, cette distillation étant réalisée de sorte que des composants organiques de point d'ébullition faible, c'est-à-dire des composés et des mélanges azéotropiques de composés dont les points d'ébullition se situent en dessous de celui du benzène à pression normale (1 013 mbar), soient séparés du benzène et du nitrobenzène, et qu'un mélange de benzène et de nitrobenzène soit ainsi obtenu, qui
f) est recyclé dans l'étape b).

2. Procédé selon la revendication 1, selon lequel l'étape d) est réalisée à une pression absolue de 900 mbar à 980 mbar.

3. Procédé selon l'une quelconque des revendications 1 ou 2, selon lequel du nitrobenzène purifié issu de l'étape c) est utilisé en tant que liquide de lavage à l'étape d) .

4. Procédé selon la revendication 3, selon lequel la distillation à l'étape c) est réalisée à une pression absolue à la tête de la colonne de distillation de 220 mbar à 480 mbar et à une température dans le fond de la colonne de distillation de 100 °C à 200 °C.

5. Procédé selon la revendication 3, selon lequel la distillation à l'étape c) est réalisée à une pression absolue à la tête de la colonne de distillation de 270 mbar à 430 mbar et à une température dans le fond de la colonne de distillation de 120 °C à 190 °C, et selon lequel le nitrobenzène purifié utilisé à l'étape d) en tant que liquide de lavage présente une teneur en benzène < 20 ppm, par rapport à la masse totale du nitrobenzène purifié.

6. Procédé selon la revendication 3, selon lequel la distillation à l'étape c) est réalisée à une pression absolue à la tête de la colonne de distillation de 320 mbar à 380 mbar et à une température dans le fond de la colonne de distillation de 160 °C à 180 °C, et selon lequel le nitrobenzène purifié utilisé à l'étape d) en tant que liquide de lavage présente une teneur en benzène < 10 ppm, par rapport à la masse totale du nitrobenzène purifié.

7. Procédé selon l'une quelconque des revendications 3 à 6, selon lequel le nitrobenzène issu de l'étape c) utilisé en tant que liquide de lavage à l'étape d) a une température de 20 °C à 75 °C.

8. Procédé selon l'une quelconque des revendications 1 à 7, selon lequel la colonne d'absorption est exploitée de sorte que les vapeurs gazeuses qui la quittent aient une température de 20 °C à 75 °C.

9. Procédé selon l'une quelconque des revendications 1 à 8, selon lequel le distributeur de liquide utilisé à l'étape d) comprend 60 à 120 emplacements d'égouttement par mètre carré.
